# EUROPEAN PATENT SPECIFICATION

(11) **EP 3 653 058 B1**
(45) Date of publication and mention of the grant of the patent: **07.09.2022**
(21) Application number: 17917824.9
(22) Date of filing: 12.07.2017
(51) Int. Cl.: A21D 8/04, A21D 2/26, A21D 10/00

(54) **METHOD FOR DEGRADATION OF GLIADIN TO OBTAIN GLUTEN-FREE FLOUR**
VERFAHREN ZUM ABBAU VON GLIADIN ZUR GEWINNUNG VON GLUTENFREIEM MEHL
PROCÉDÉ DE DÉGRADATION DE GLIADINE POUR OBTENIR UNE FARINE SANS GLUTEN

(43) Date of publication of application: 20.05.2020
(73) Proprietor: González de La Torre, Javier, 45653 Tlajomulco de Zúñiga, Jalisco (MX)
(72) Inventor: PEDROZA ISLAS, Ruth, México 03570 (MX)
(74) Representative: Regimbeau
(86) International application number: PCT/IB2017/054218
(87) International publication number: WO 2019/012312

(56) References cited:
- WO-A1-00/10395
- WO-A1-2014/033765
- WO-A1-2014/072758
- WO-A2-2010/073283
- US-A1- 2015 351 415
- Carlo G. Rizzello, Maria De Angelis, Raffaella Di Cagno, Alessandra Camarca, Marco Silano, Ilario Losito, Massimo De Vincenzi, Mar: "Highly Efficient Gluten Degradation by Lactobacilli and Fungal Proteases during Food Processing: New Perspectives for Celiac Disease", Applied and Environmental Microbiology, vol. 73, no. 14, 11 July 2007 (2007-07-11) , pages 4499-4507, XP055539589, DOI: 10.1128/AEM.00260-07
- Rizzello CG, Curiel JA, Nionelli L, Vincentini O, Di Cagno R, Silano M, Gobbetti M, Coda R.: "Use of fungal proteases and selected sourdough lactic acid bacteria for making wheat bread with an intermediate content of gluten.", Food Microbiology, vol. 37, 5 July 2013 (2013-07-05), pages 59-68, XP028770938, DOI: 10.1016/j.fm.2013.06.017
- Alvarez-Sieiro P, Redruello B, Ladero V, Cañedo E, Martin MC, Fernández M, Alvarez MA: "Solubilization of gliadins for use as a source of nitrogen in the selection of bacteria with gliadinase activity.", Food Chemistry, vol. 168, 24 July 2014 (2014-07-24), pages 439-444, XP055668239, DOI: 10.1016/j.food.2014.07.085
- Maria De Angelis, Angela Cassone, Carlo G. Rizzello, Francesca Gagliardi, Fabio Minervini, Maria Calasso, Raffaella Di Cagno, Rugg: "Mechanism of degradation of immunogenic gluten epitopes (Triticum turgidum L. var. durum) by sourdough lactobacilli and fungal proteases", Applied and Environmental Microbiology, vol. 76, no. 2, 2010, pages 508-518, XP009154621, DOI: 10.1128/AEM.01630-09

## Description

### FIELD OF THE INVENTION

The present invention relates to processes for manufacturing gluten-free flours, more specifically, a process for degradation of gliadin to obtain a gluten-free flour.

### BACKGROUND OF THE INVENTION

Celiac disease is a genetic disorder of the immune system that is induced by the ingestion of gluten, which is a protein found in wheat, rye, and barley. Gluten is poorly digested in the human upper gastrointestinal tract. Gluten consists of two components, gliadin and glutenin. Gliadin is alcohol-soluble and contains the greatest amount of toxic components for people with celiac disease (Green, P-H-R- and Cellier, C. 2007. Celiac disease. N. Engl. J. Med.; 357:1731-1743).

When a patient with celiac disease eats some food containing gluten, their immune system responds in such a way that damages or destroys the intestinal villi, which causes the nutrients in the food to not be absorbed, thus causing poor nutrition. Symptoms of celiac disease vary depending on the patient's age, with diarrhea, abdominal distention, vomiting, and weight loss being the most common in children, while in adults the prevailing symptoms are iron-deficiency anemia, fatigue, bone pain, arthritis, osteoporosis, among others.

Until recently, celiac disease was considered to be a rare disease, but it is currently one of the most common intolerances, having a worldwide prevalence of 1 to every 150 newborns, and it is estimated that only 9% are diagnosed. According to the statistics provided by the National Institute of Medical Sciences and Nutrition Salvador Zubirán, in Mexico, there are approximately 2.6 million potential people with celiac disease.

Nowadays, the only accepted treatment for celiac disease is to follow a lifelong gluten-free diet. According to the Codex Alimentarius, a food is considered to be gluten-free when its gluten concentration is less than 20 ppm. However, this represents negative nutritional implications such as lower polysaccharide intake and thus reduced energy intake, reduced gut microbiota beneficial to human health, and increased presence of opportunistic pathogens. The beneficial gut microbiota reduced by gluten-free diets negatively affects immunostimulatory activity, and the production of anti-inflammatory compounds is decreased.

For this reason, there is a need for gluten-free products suitable for celiac disease patients, and accordingly, which can allow the patients to improve their diet by being able to have a greater variety of food goods.

One of the most important products in the human daily diet is bread made mainly from wheat flour. The properties of wheat dough depend primarily on gluten proteins. In recent years, various treatments have been applied to improve the quality of these proteins or to degrade them, thus obtaining bakery products that can be consumed by people with celiac disease.

A recently developed treatment to improve the quality of bread is the production of sourdoughs. Said doughs are obtained from a mix of flour and water that is fermented by yeasts and lactic acid bacteria (LAB) generally belonging to the genus *Lactobacillus.* It has been discovered that LABs used in fermentation to obtain sourdough allows the modification of gluten during bread making, which allows the elimination of protein fractions that are toxic to patients with celiac disease. One of the most harmful protein fractions for people suffering from this condition is the gliadin fraction, which can also be eliminated by using LAB.

During fermentation, the proteolytic system of LABs releases low molecular weight peptides and amino acids that promote the metabolic activity of microorganisms, contributing to improved taste formation and reduced allergenic peptide content, which provides great hope to celiac disease patients who are mainly sensitive to the gliadin fraction. Studies conducted on the sourdough bread production showed that LABs have the capacity to hydrolyze the wheat gliadin fraction under specific processing conditions (long-term and semi-liquid fermentation), (Di Cagno, R., De Angelis, M., Auricchio, S., Greco, L., Clarke, C., De Vincenzi, M., & Minervini, F. (2004). Sourdough bread made from wheat and nontoxic flours and started with selected lactobacilli is tolerated in celiac sprue patients. Applied and environmental microbioiogy, 70(2), 1088-1096; Gobbetti, M., De Angelis, M., Corsetti, A., & Di Cagno, R. (2005). Biochemistry and physiology of sourdough lactic acid bacteria. Trends in Food Science & Technology, 16(1), 57-69.; Gobbetti, M., Rizzello, C. G., Di Cagno, R., & De Angelis, M. (2007). Sourdough lactobacilli and celiac disease, Food microbiology, 24(2), 187-196,).

However, not all lactic acid bacteria can reduce the residual gluten concentration to doses that can be tolerated by gluten-intolerant people (celiac disease patients), thus it is necessary to select the type of LAB and find a suitable combination therebetween.

Proteases also have the capacity to degrade gluten fractions. This is why proteases are used to assist in the hydrolysis of gluten.

In the paper by Rizzello, C.G. et al. (2007). Highly efficient gluten degradation by lactobacilli and fungal proteases during food processing: new perspectives for celiac disease, Applied and environmental microbiology, 73(14), 4499-4507, mixtures of non-commercial *Lactobacilli* strains (previously selected based on their capacity to hydrolize gliadins) with fungal proteases in different combinations were used in sourdoughs to remove toxicity from wheat flour during a relatively long fermentation. It was observed that the kinetics of hydrolysis by *Lactobacilli* was highly efficient in hydrolyzing albumins, globulins and gliadins, but 20% glutenins remained in the sourdough. In Rizzello et al.: "Use of fungal proteases and selected sourdough lactic acid bacteria for making wheat bread with an intermediate content of gluten"; Food Microbiology; 2014; 37; pages 59-68, it is disclosed that lactic acid bacteria are used for sourdough fermentation at 30°C (see e.g., page 60 of D2) under stirring and varying parameters such as the concentration of fungal proteases.

Likewise, U.S. Patent Application Publication US 2008/0131556 discloses a mixture of at least six commercially available species of LAB and/or Bifidobacteria. This mixture can be used in the preparation of sourdoughs. When a sufficient amount of microbial proteases commonly used in bakery are added to these formulations, the gluten is degraded, but not sufficiently, since the fermented sourdough has a gluten concentration of about 200 ppm, which may not be suitable for the consumption of a celiac disease patient. Furthermore, the mixture which achieves this degradation is complex because it is necessary to use a large number of species, since a greater degradation of gliadins can be observed when more LAB species are used.

International Publication WO 2010/073283 discloses a mixture comprising two types of LAB, in combination with one or more fungal proteases. The LABs used are strains specifically developed to have a higher degree of gluten degradation. By means of said combination of LAB and proteases, in specific amounts and under certain reaction conditions, gliadin and glutenin traces were reduced to the extent that they could not be detected, and a residual gluten concentration of less than 20 ppm was achieved. At the end of the gluten degradation process, a gluten-free flour is obtained for use in bakery goods. However, the problem in this case is that the microorganisms used are not commercially available.

Also, International Publication WO 2014/072758 shows a microencapsulated bacterial consortium for the degradation of gluten into sourdoughs. Said invention has the advantage of reducing the difficulties in preparing the inoculum whenever it is required, reducing the risks of contamination and on viability of lactic acid cultures, promoting process efficiency for the degradation of wheat doughs, but as in the previous case, it requires the use of specific microorganisms adapted to the fermentation process.

As can be seen, bacterial cultures already known in the prior art for gluten degradation have certain disadvantages such as the use of complex LAB mixtures (of at least six species) in the case of commercially available strains, or rather, the use of specifically selected and designed strains, which implies the need to activate, reproduce, wash and add the culture in suspension (inoculum) to the dough, requiring daily preparation thereof and highly specialized handling to avoid contamination, keep it active in the same growing stage and in the suitable proportion between LAB species and in a sufficient amount, which represents a latent risk of contamination and finally, the treatment of a dough by means of microencapsulated inoculums, which implies additional steps to baking process.

Also, in the case of the process in which a baking flour can be obtained as the final product, it requires very specific bacterial consortia to be used, which, as previously indicated, involve activation, reproduction and washing of the cultures. Another disadvantage of these types of cultures is that they involve a very complicated process to be developed, which means a considerable increase in the cost of flour production, which makes it unfeasible.

Thus, in the state of the art, there is currently a need to develop a gliadin degradation process which is efficient, uses commercially available microorganisms, and by which a flour having very low content of gliadins and therefore gluten, can be obtained.

### OBJECTS OF THE INVENTION

Considering the problems and disadvantages of the state of the art mentioned above, it is an object of the present invention to provide a process for degradation of gliadins in flour for bread making by means of enzymatic hydrolysis and microbial fermentation.

### SUMMARY OF THE INVENTION

The process for degradation of gliadin in flour for bread making of the present invention solves the problems and disadvantages mentioned in the state of the art.

The present invention relates to a process for degradation of gliadin in flour for bread making comprising: a step of mixing the flour with water; at least one step of enzymatic hydrolysis to hydrolyze gliadin from the mixture obtained in the mixing step; at least one step of fermenting the mixture obtained in the enzymatic hydrolysis step by using at least one microorganism under controlled-pH conditions; wherein the microorganism is selected from Bacillus amyloliquefaciens, Lactobacillus brevis, Lactobacillus delbrueckii, Lactobacillus reuteri, Lactobacillus helveticus or mixtures thereof; and a step of drying to obtain a gliadin-free

flour. The present description is defined by the appended claims.

### DESCRIPTION OF DRAWINGS

Figure 1 shows a chromatogram of flour treated by means of a 5-hour proteolysis and lactobacillus fermentation.
Figure 2 shows a chromatogram of flour treated by means of a 10-hour proteolysis and lactobacillus fermentation.
Figure 3 shows a chromatogram of flour treated by means of fermentation by *L. johnsonii* for 24 hours.
Figure 4 shows a chromatogram of flour treated by means of a proteolysis using HT Proteolytic 200^{®} for 10 hours.
Figure 5 shows a chromatogram of flour treated by means of a proteolysis using Harizyme G^{®} for 10 hours.
Figure 6 shows a chromatogram of flour treated by means of a 5-hour proteolysis, lactobacillus fermentation, and a proteolysis using protease HT Proteolitic 200^{®} for 5 hours.
Figure 7 shows a concentration of gliadin in flour fermented by *L. sanfranciscensis.*

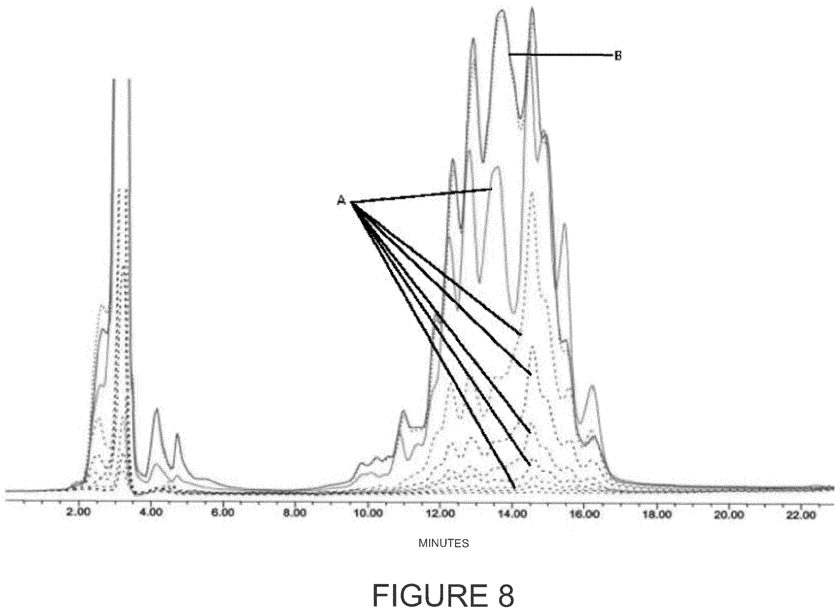

Figure 8 shows a concentration of gliadin in flour fermented by *L. brevis.*

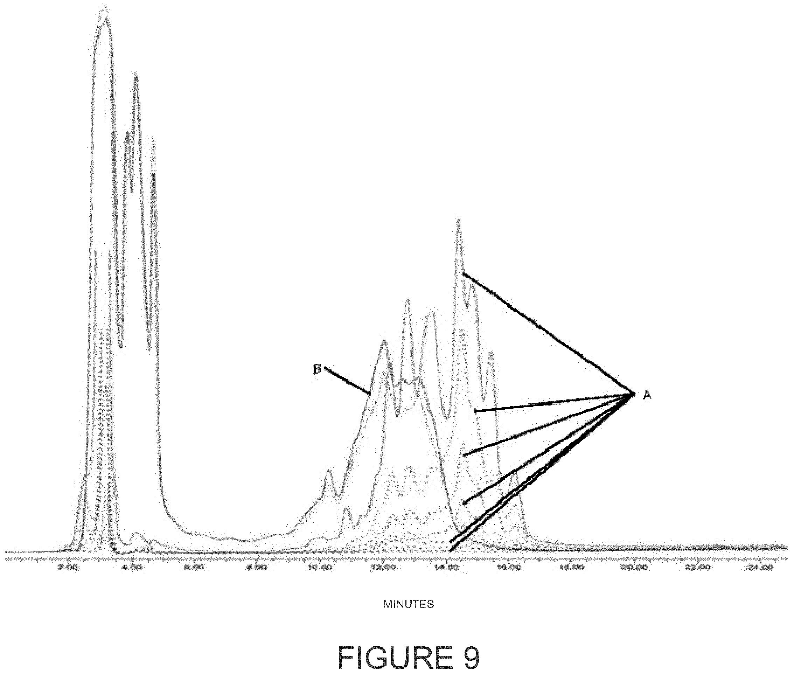

Figure 9 shows a concentration of gliadin in flour fermented by *Bacillus amyloliquefaciens.*
Figure 10 shows a concentration of L-tyrosine during an enzymatic proteolysis using protease N-1000^{®}.
Figure 11 shows a concentration of L-tyrosine during an enzymatic proteolysis using protease HT Proteolitic 200^{®}.
Figure 12 shows a liquid chromatogram of the flour treated by using a process comprising two enzymatic hydrolysis steps and two fermenting steps.

### DETAILED DESCRIPTION OF THE INVENTION

As mentioned in the background, the degradation of gluten fractions, especially gliadins, is highly important for making products suitable for celiac disease patients. To solve this problem, it has been found that by means of a process consisting of at least one step of enzymatic hydrolysis and at least one step of fermentation using lactobacilli, the total degradation of gliadin contained in baking flours is achieved.

It has been observed that the acidification process of the medium that takes place when treating flours by fermentation and enzymatic hydrolysis negatively affects the degree of gliadin degradation in the flour because the action of microorganisms and enzymes is inhibited. This is why in order to obtain a flour with optimally degraded gliadins, not only must bacterial consortia and reaction conditions be carefully selected, but also each of these processes must be carried out in a specific order that optimizes the microorganism activity and that avoids the enzymatic inhibition.

Thus, the process for degradation of gliadin in flour for bread making of the present invention solves the problems and disadvantages mentioned in the state of the art.

In accordance with the foregoing, the present invention relates to a process for degradation of gliadin in flour for bread making comprising: a step of mixing the flour with water; at least one step of enzymatic hydrolysis to hydrolize gliadin from the mixture obtained in the mixing step; at least one step of fermenting the mixture obtained in the enzymatic hydrolysis step by using at least one microorganism under controlled-pH conditions; and, a step of drying to obtain a gliadin-free flour.

The flours for bread making are all of those having the suitable properties for making bread goods. They are characterized by having a high gluten content, which allows said flour to have a good water absorption capacity, cohesiveness, viscosity, and elasticity. Some examples of flours for bread making are flours derived from wheat, rye, barley, oatmeal, and triticale.

The first step of said process comprises mixing flour for bread making with water.

In a preferred embodiment of the present invention, fungal enzymes are preferably used in the enzymatic hydrolysis step. More preferably, enzymes from *Aspergillus niger* and/or *Aspergillus oryzae.* Said enzymes are preferably present in a 1:1 ratio and are added to the mixture in a proportion from between 0.01 and 0.1% by weight relative to the suspension. The enzymatic hydrolysis step is preferably carried out at a temperature from between 35 and 37 °C, under constant stirring. The hydrolysis time is preferably from between 4 and 8 hours. In this first step, a degradation of about 88% gliadin present in the initial flour is achieved.

As previously stated, the medium tends to be acidified by the enzymatic hydrolysis, and it is known that the enzyme activity tends to decrease at an acidic pH. That is why that in an optional embodiment of the present invention, after the hydrolysis step is completed, a pH adjustment is carried out. Preferably, a pH between 6.5 and 8 is sought. To carry out said pH adjustment, a base, preferably K₂CO₃, is added in an amount sufficient to reach the pH required.

During the hydrolysis step described, the hydrolysis of gliadin and the breakdown of target peptides present in the gluten molecules are achieved. Peptides and amino acids generated by the enzymatic hydrolysis process can be degraded efficiently by some LABs. That is why a subsequent step of the process for degradation of gliadin is a step of fermenting the mixture obtained in the enzymatic hydrolysis step by using at least one LAB under controlled-pH conditions.

As to the step of fermenting, it is carried out by using *Bacillus amyloliquefaciens, Lactobacillus brevis, Lactobacillus delbrueckii, Lactobacillus reuterii, Lactobacillus helveticus* or mixtures thereof as the microorganisms. The microorganism used is preferably added to the mixture in an amount from between 10³ and 10⁹ CFU/g of flour. Preferably, the step of fermenting is carried out at a temperature of from 36 to 38 °C and for a period from between 4 and 8 hours. In the mentioned step of fermenting, up to an additional 10% of initial gliadins can be degraded, with the remaining flour having about 2% of gliadins.

As in the hydrolysis step, during the step of fermenting, a considerable decrease in pH is observed, thus in an optional embodiment of the present invention, a second pH adjustment is carried out. Preferably a pH between 6.5 and 8 is sought. To carry out said pH adjustment, a base, preferably K₂CO₃, is added in an amount sufficient to reach the pH required.

To degrade the remaining 2% of gliadins, a second hydrolysis step may be implemented. Thus, in a further preferred embodiment of the present invention, a second enzymatic hydrolysis step is carried out by using preferably fungal enzymes. More preferably enzymes from *Aspergillus niger* and/or *Aspergillus oryzae.* Said enzymes are preferably present in a 1:1 ratio and are added to the mixture in a proportion from between 0.02 and 0.08% by weight relative to the suspension. The second enzymatic hydrolysis step is preferably carried out at a temperature from between 35 and 37 °C, under constant stirring. The hydrolysis time is preferably from 2 to 4 hours.

Additionally, after the second hydrolysis step, a second fermenting step can be carried out to achieve a maximum possible degradation of gliadins. Accordingly, in a preferred embodiment of the present invention, a second step of fermenting the mixture obtained in the second enzymatic hydrolysis step is carried out by using at least one microorganism under controlled-pH conditions. Preferably, the second fermenting step is carried out by using *Bacillus amyloliquefaciens, Lactobacillus brevis, Lactobacillus delbrueckii, Lactobacillus reuterii, Lactobacillus helveticus* or mixtures thereof as the microorganisms. Preferably, a mixture of *Bacillus amyloliquefaciens* and at least two lactobacilli selected from: *Lactobacillus brevis, Lactobacillus delbrueckii, Lactobacillus reuterii,* and *Lactobacillus helveticus* is used. Preferably, the microorganisms are present in a 1:1:1 ratio. Preferably, the microorganisms are added to the mixture in an amount from between 10³ and 10⁹ CFU/g of flour. Preferably, the second fermenting step is carried out at a temperature from between 35 and 37 °C, under constant stirring and for a period of time from between 4 and 12 hours.

Finally, to obtain a final product that can be used in the production of bakery products, the process includes an additional step of drying to obtain a gliadin-free flour.

In a preferred embodiment, the drying step is a spray drying process. Preferably, in the spray drying, inlet air is at a temperature from between 150 and 180 °C and outlet air is at a temperature from between 70 and 90 °C.

The present invention will be better understood from the following examples, which are presented for illustrative purposes only to allow a full understanding of the preferred embodiments of the present invention, without implying that there are no other non-illustrated embodiments that can be put into practice based on the detailed description set forth above.

### EXAMPLE 1

Several studies were conducted to observe the gliadin degradation by various methods.

In the first study, a process consisting of a proteolysis step and a fermentation step was carried out. For this study, a flour-in-water suspension was made, and said suspension was kept under constant stirring at a temperature of 37 °C. For the proteolysis step, 1 g enzyme HT Proteolytic^{®} per kg of flour was added and left to hydrolize for 5 hours. Subsequently, 10⁹ CFU of the lactobacilli *Lactobacillus brevis* and *Lactobacillus sanfranciscensis* were added for the fermentation step, which lasted 9 hours.

In the second study, the previous assay was repeated, but the enzymatic proteolysis time was changed from 5 to 10 hours.

In the third study, a fermentation process was carried out, for which a flour-in-water suspension was made, and 10⁹ CFU of *L. johnsonii* were added to said suspension. The suspension was kept under constant stirring at a temperature of 37 °C for 24 hours.

The fourth study consisted of a proteolysis process, for which a suspension of 33.3 g flour and 130 ml water was made, and 1 g enzyme HT Proteolitic 200^{®} per kg of flour was added to said suspension. Said suspension was kept under constant stirring at 37 °C for 10 hours.

In the fifth study, a proteolysis process was carried out, for which a suspension of 33.3 g flour and 130 ml water was made, and 1.2 g enzyme Harizyme G^{®} per kg of flour were added to said suspension. The suspension was kept under constant stirring at 37 °C for 10 hours.

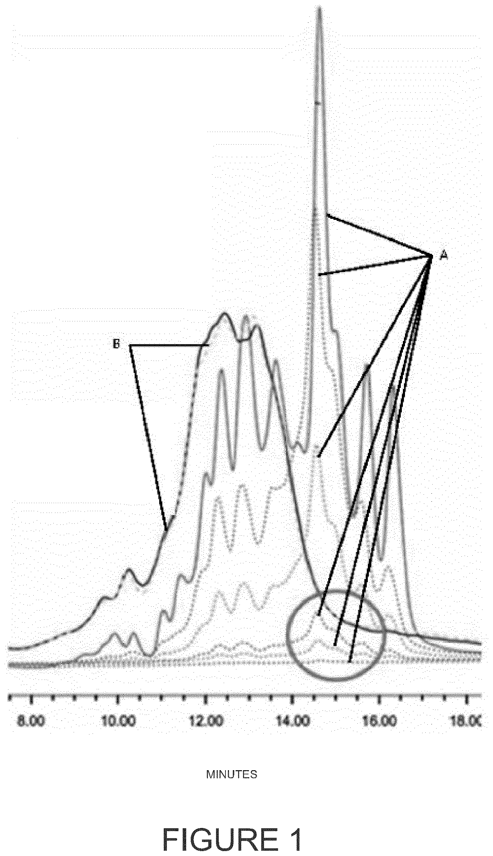

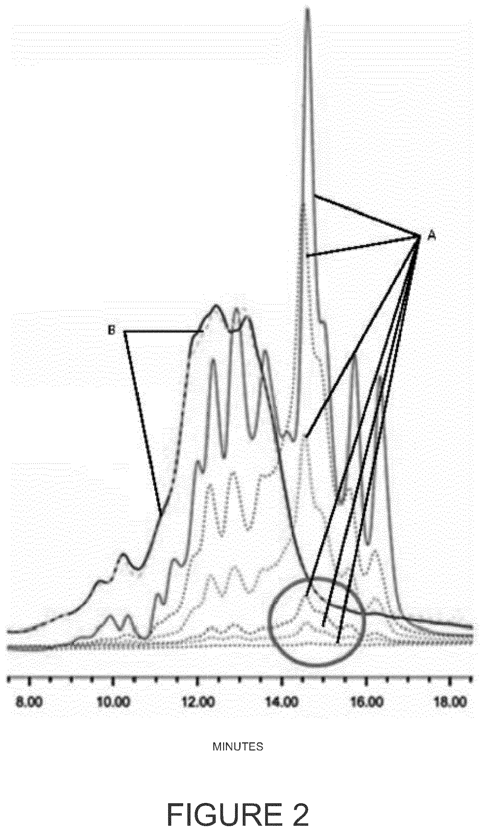

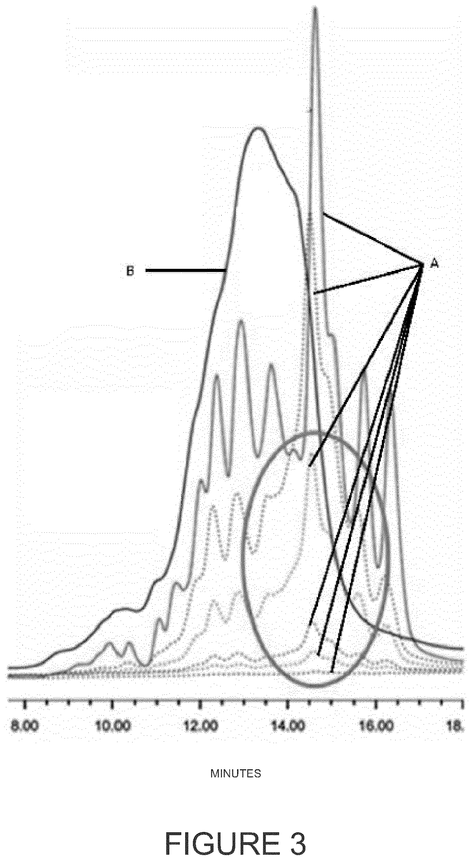

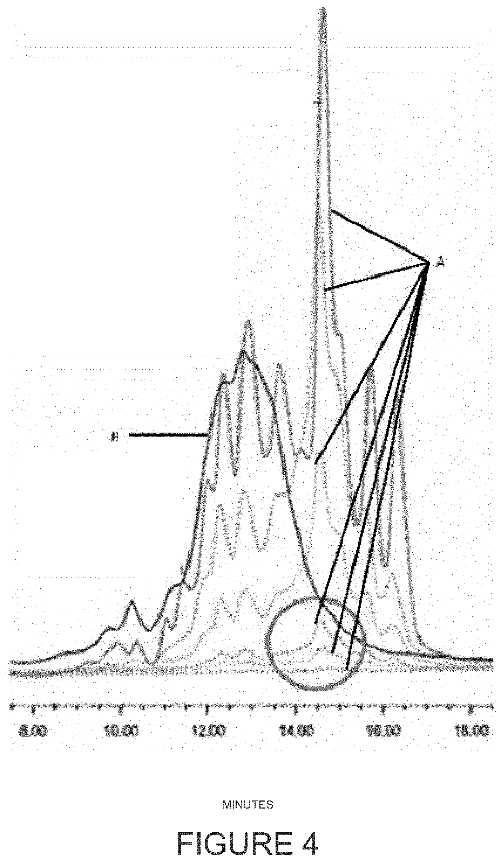

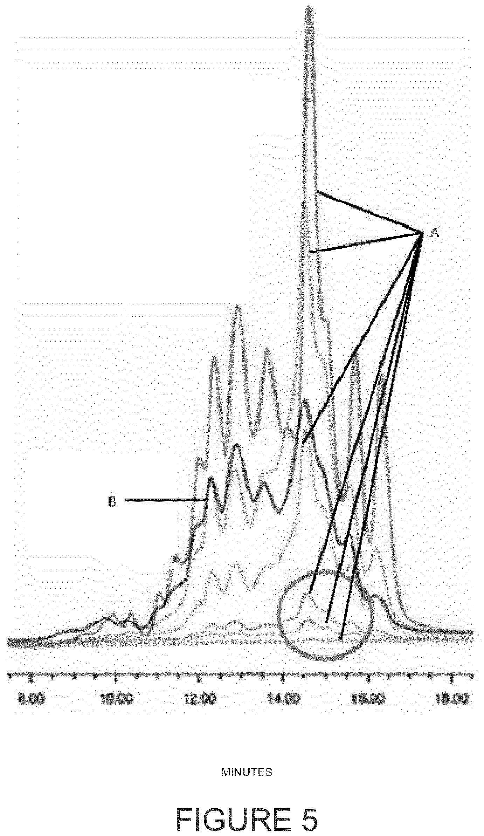

At the end of each experiment, the flour was analyzed by liquid chromatography. The results can be seen in Table 1. In each of Figures 1 to 5, a liquid chromatogram of the flour Hoja de Plata^{®} is indicated by a letter "A", and a chromatogram of the flour treated by each of the methods of the present study is indicated by the letter "B". Also, the position of the chromatogram that shows the analyzed gliadin content is indicated by a circle in each figure.

**Table 1**

| Experiment | Result | Chromatogram |
|---|---|---|
| Proteolysis for 5h and lactobacillus fermentation | A considerable decrease of gliadin fractions was observed although not completely degraded | Figure 1 |
| Proteolysis for 10h and lactobacillus fermentation | A considerable gliadin degradation was observed, however, only 20% reduction compared to the previous proteolysis treatment | Figure 2 |
| Fermentation by *L. johnsonii* for 24h | A decrease of gliadin fractions was observed, however, an increase in higher molecular weight peptides was detected | Figure 3 |
| Proteolysis using HT Proteolitic 200^{®} for 10h | A considerable gliadin decrease was observed as well as a decrease in higher molecular weight peptides detected in previous experiments | Figure 4 |
| Proteolysis using Harizyme G^{®} for 10h | A profile very similar to that of the standard flour Hoja de Plata was observed, which indicates that gliadin degradation was minimal | Figure 5 |

From the results shown in Table 1 and their respective Figures, it is concluded that the most efficient gluten degradation processes are those wherein a proteolysis was carried out followed by a step of lactobacillus fermentation.

### EXAMPLE 2

An assay was carried out to observe gliadin degradation by a process comprising a 5-h proteolysis step using enzyme N1000^{®}, a fermentation step using lactobacilli and an additional proteolysis step using HT Proteolitic 200^{®}.

For this assay, a first experiment was carried out wherein a flour-in-water suspension was made, and 0.4 g enzyme N-1000^{®} per kg of flour was added to said suspension. The suspension was kept under constant stirring for 5 hours at a temperature of 37 °C. Subsequently, 10⁹ CFU *Lactobacillus brevis* and *Lactobacillus sanfranciscensis* were added and kept under constant stirring for 9 hours at a temperature of 37 °C. Finally, 1 g enzyme HT Proteolitic 200^{®} per kg of flour was added and the suspension was kept under constant stirring for 5 hours at a temperature of 37 °C.

The experiment was repeated but changing only the second proteolysis time to 10 hours.

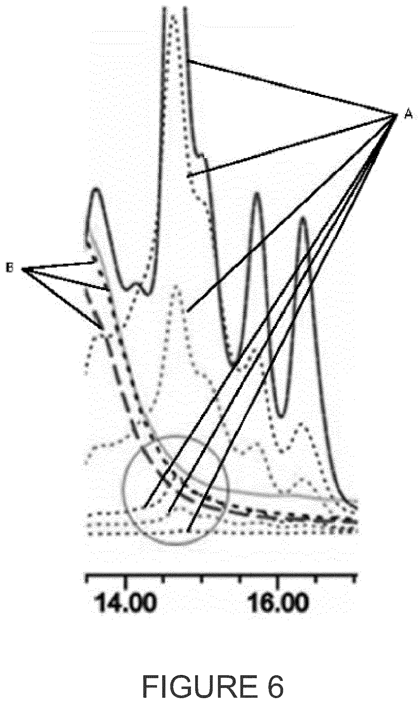

From said experiment, the chromatogram of Figure 6 was obtained wherein a chromatogram of the flour Hoja de Plata^{®} at different concentrations is indicated by the letter "A" and a chromatogram of the flour treated by the present method is indicated with the letter "B". In the same figure, the position of the chromatogram that shows the gliadin content of the analized flour is indicated by a circle.

From Figure 6, it can be seen that after all the process steps were carried out, it was possible to degrade gliadin to about 75 ppm. Figure 6 also shows that the results of the first experiment were very similar to those of the second experiment.

### EXAMPLE 3

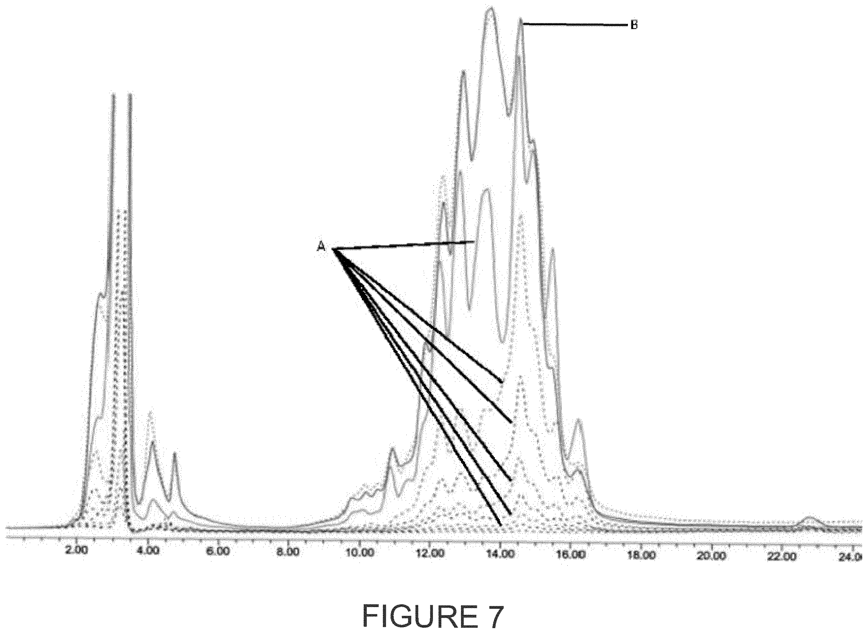

Three fermentation processes were carried out. In each process, a flour-in- water suspension was made, and 10⁹ CFU of a microorganism were added to said suspension. In the first experiment, the microorganism was *L. brevis;* in the second experiment, the microorganism selected was *L. sanfranciscensis;* and in the third experiment it was B. *amyloliquefaciens.* Each fermentation lasted 24 hours at 37 °C under constant stirring. Subsequently, the gliadin degraded was quantified by liquid chromatography. The results can be seen in Figures 7 for *L. sanfranciscensis,* 8 for *L. brevis,* and 9 for *B. amyloliquefaciens.* Wherein "A" lines indicate gliadin standards and "B" line indicates the gliadin concentration in flour fermented by each of the microorganisms.

As can be seen, the best microorganism for degrading gliadin is *B. amyloliquefaciens.*

### EXAMPLE 4

A study was conducted to determine the operating time required to carry out enzymatic hydrolysis. For this study, the tyrosine release was quantified during two wheat gluten hydrolysis processes, each with a different protease. The purpose of this experiment was to determine the hydrolysis time required for tyrosine to reach steady state, that is, when its stops being generated.

For these experiments, the tyrosine content in flour suspensions was quantified in the hydrolysis process every 15 to 30 minutes. For the first experiment, a suspension of 4.3 g flour in 130 ml water was used, and 0.4 g protease N-1000^{®} was added to said suspension. For the second experiment, a suspension of 4.3 g gluten in 130 ml water was used, and 1 g protease Proteolitic 200^{®} HT was added to said suspension.

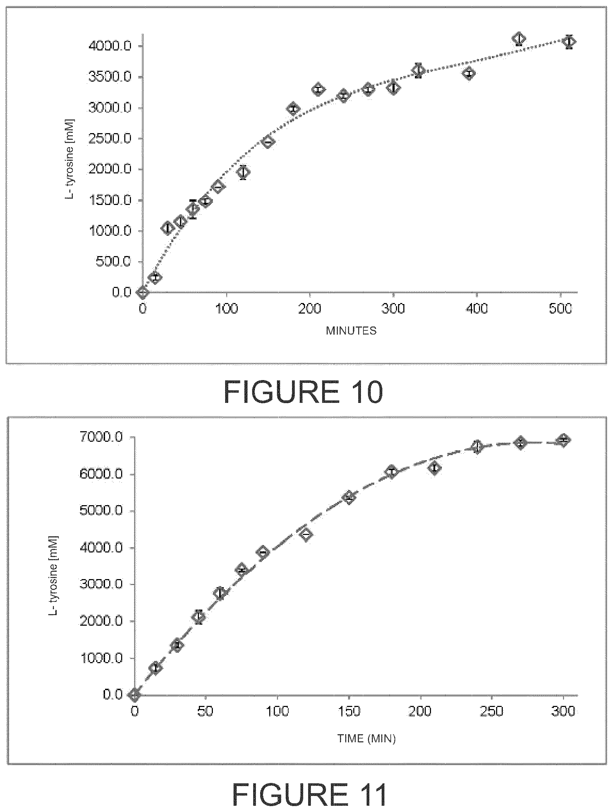

The results obtained can be seen in Figure 10 for protease N-1000^{®} and in Figure 11 for protease Proteolitic 200^{®} HT, respectively. In these Figures, the tyrosine concentration is shown over time and it can be clearly seen that on average, the tyrosine concentration in the suspensions reaches the steady state between 4 and 8 hours of hydrolysis.

### EXAMPLE 5

An assay was carried out under conditions equivalent to those mentioned in the previous examples to determine the gliadin concentration present in the flour after being treated by the gliadin degradation process of the present invention.

For this study, a flour-in-water suspension was made, it was first treated by means of an enzymatic hydrolysis step using enzymes ENZECO^{®} PROTEASE FNP and ENZECO^{®} FUNGAL PROTEASE, then by a fermentation step using *Bacillus amyloliquefaciens,* followed by another enzymatic hydrolysis step using proteases ENZECO^{®} PROTEASE FNP and ENZECO^{®} FUNGAL PROTEASE, and finally by a second fermentation using *Bacillus amyloliquefaciens* in combination with *L. brevis* and *L. delbrueckii.* All of the steps were carried out at 37 °C under constant stirring.

Hydrolized flour samples were obtained by the quartering method. For each replica, 200 mg of sample were taken and placed in Falcon tubes with a glass bead to improve extraction.

Protein extraction was carried out by performing three washes with a 70% ethanolic solution. Each wash was performed by adding 3 ml ethanolic solution to sample and glass bead-containing tubes and they were stirred for one hour in a 70- RPM rotary incubator. After centrifuging the sample, it was re-suspended with solvent using a vortex. The soluble part in the solvent was decanted after centrifuging the tube for 10 minutes at 2500 RPM, and finally the extract was placed in another Falcon tube wherein the three washes were combined.

3 ml extract from each sample were filtered through a 0.22-µm membrane to analyze the gliadin concentration by HPLC.

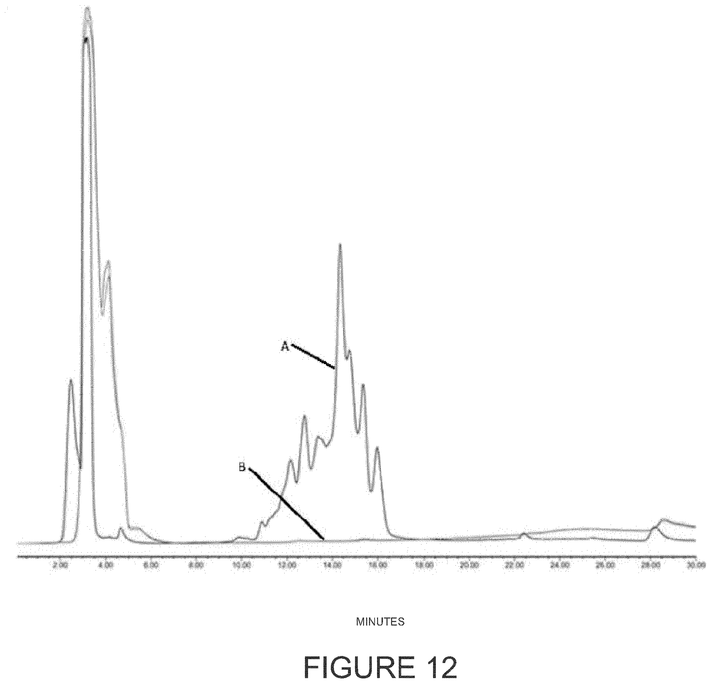

The sample extractions were passed through an HPLC equipment at a temperature of 50 °C using two mobile phases, the first named solution A which consisted of a mixture of 99% water and 1% water with 0.001% acid trifluoroacetic. The mobile phase flow was 0.6ml/min with a gradient of 27% B to 50% B for the first 20 minutes and finally 50% B to 75% B for up to 30 minutes, after the injection of a sample, a wash with phase A was performed for 10 minutes. 100µm sample or phase A were injected during the analysis and washed respectively. Peptide signals present at 210 nm were detected by using a UV detector, which can be seen in Figure 12, wherein a liquid chromatogram of a standard flour is indicated by the letter "A" and a liquid chromatogram of a flour treated by means of the disclosed method is indicated by the letter "B".

From Figure 12, it can be seen that the fractions corresponding to the different gliadins (RF between 12 and 17 minutes) apparently disappear in the samples having flour treated by the method of the present invention.

In accordance with the foregoing, it shall be apparent to a person skilled in the art that the preferred embodiment of the process for degradation of gliadin in flour for bread making illustrated above is set forth for illustrative purposes only but not limited to the present invention, since a person skilled in the art can make numerous variations thereto, provided they are designed according to the principles of the present invention.

Accordingly, the present invention includes all of the embodiments that a person skilled in the art can pose from the concepts contained in the present specification, in accordance with the following claims.

## Claims

1. A process for degradation of gliadin in flour for bread making, **characterized in that** it comprises: a step of mixing the flour with water; at least one step of enzymatic hydrolysis to hydrolyze gliadin from the mixture obtained in the mixing step; at least one step of fermenting the mixture obtained in the enzymatic hydrolysis step by using at least one microorganism under controlled-pH conditions; wherein the microorganism is selected from *Bacillus amyloliquefaciens, Lactobacillus brevis, Lactobacillus delbrueckii, Lactobacillus reuteri, Lactobacillus helveticus* or mixtures thereof; and, a step of drying to obtain a gliadin-free flour.

2. The process for degradation of gliadin in flour for bread making according to claim 1, further **characterized in that** fungal enzymes are used in the enzymatic hydrolysis step, preferably enzymes from *Aspergillus niger* and/or *Aspergillus oryzae* in a 1:1 ratio.

3. The process for degradation of gliadin in flour for bread making according to claim 2, further **characterized in that** the enzymes are added to the mixture in a proportion from between 0.01 and 0.1% by weight relative to the suspension.

4. The process for degradation of gliadin in flour for bread making according to claim 1, further **characterized in that** the enzymatic hydrolysis step is carried out at a temperature from between 35 and 37 °C, preferably under constant stirring.

5. The process for degradation of gliadin in flour for bread making according to claim 1, further **characterized in that** hydrolysis time is from between 4 and 8 hours.

6. The process for degradation of gliadin in flour for bread making according to claim 1, further **characterized in that** after the hydrolysis step is completed, a pH adjustment is carried out, preferably the pH being adjusted to between 6.5 and 8.

7. The process for degradation of gliadin in flour for bread making according to claim 6, further **characterized in that** the pH adjustment is carried out by adding a base, preferably K₂CO₃.

8. The process for degradation of gliadin in flour for bread making according to claim 1, further **characterized in that** the microorganism used is added to the mixture in an amount from between 10³ and 10⁹ CFU/g of flour.

9. The process for degradation of gliadin in flour for bread making according to claim 1, further **characterized in that** the fermenting step is carried out at a temperature of from 36 to 38 °C, preferably for a period from between 4 and 8 hours.

10. The process for degradation of gliadin in flour for bread making according to claim 1, further **characterized in that** after the fermenting step is completed, a second pH adjustment is carried out, preferably the pH being adjusted to between 6.5 and 8.

11. The process for degradation of gliadin in flour for bread making according to claim 10, further **characterized in that** the second pH adjustment is carried out by adding a base, preferably K₂CO₃.

12. The process for degradation of gliadin in flour for bread making according to claim 1, further **characterized in that** it comprises a second enzymatic hydrolysis step.

13. The process for degradation of gliadin in flour for bread making according to claim 12, further **characterized in that** fungal enzymes are used in the second enzymatic hydrolysis step, preferably enzymes from *Aspergillus niger* and/or *Aspergillus oryzae* in a 1:1 ratio.

14. The process for degradation of gliadin in flour for bread making according to claim 13, further **characterized in that** the enzymes are added to the mixture in a proportion from between 0.01 and 0.1% by weight relative to the suspension.

15. The process for degradation of gliadin in flour for bread making according to claim 13, further **characterized in that** the second enzymatic hydrolysis step is carried out at a temperature from between 35 and 37 °C, preferably under constant stirring.

16. The process for degradation of gliadin in flour for bread making according to claim 13, further **characterized in that** a second enzymatic hydrolysis time is of from 2 to 4 hours.

17. The process for degradation of gliadin in flour for bread making according to claim 1, further **characterized in that** it comprises a second fermenting step.

18. The process for degradation of gliadin in flour for bread making according to claim 17, further **characterized in that** the second fermenting step is carried out by using at least one microorganism under controlled-pH conditions, preferably the microorganism being selected from *Bacillus amyloliquefaciens, Lactobacillus brevis, Lactobacillus delbrueckii, Lactobacillus reuterii, Lactobacillus helveticus* microorganisms or mixtures thereof.

19. The process for degradation of gliadin in flour for bread making according to claim 18, further **characterized in that** a mixture of *Bacillus amyloliquefaciens* and at least two lactobacilli selected from: *Lactobacillus brevis, Lactobacillus delbrueckii, Lactobacillus reuterii,* and *Lactobacillus helveticus* is used, preferably in a 1:1:1 ratio.

20. The process for the degradation of gliadin flour for bread making according to claim 19, further **characterized in that** the microorganism is added to the mixture in an amount from between 10³ and 10⁹ CFU/g of flour.

21. The process for degradation of gliadin in flour for bread making according to claim 18, further **characterized in that** the second fermenting step is carried out at a temperature from between 35 and 37 °C, preferably under constant stirring.

22. The process for degradation of gliadin in flour for bread making according to claim 19, further **characterized in that** the second fermenting step is carried out for a period from between 4 and 12 hours.

23. The process for degradation of gliadin in flour for bread making according to claim 1, further **characterized in that** the drying step is a spray drying process.

24. The process for degradation of gliadin in flour for bread making according to claim 23, further **characterized in that** inlet air is at a temperature from between 150 and 180 °C and outlet air is at a temperature from between 70 and 90 °C in the spray drying.

## Patentansprüche

1. Verfahren zum Abbau von Gliadin in Mehl zur Brotherstellung, **dadurch gekennzeichnet, dass** es umfasst: einen Schritt eines Mischens des Mehls mit Wasser; mindestens einen Schritt einer enzymatischen Hydrolyse, um Gliadin aus dem in dem Mischschritt erhaltenen Gemisch zu hydrolysieren; mindestens einen Schritt eines Fermentierens des in dem enzymatischen Hydrolyseschritt erhaltenden Gemischs durch Verwenden von mindestens einem Mikroorganismus unter kontrollierten pH-Wert-Bedingungen; wobei der Mikroorganismus aus *Bacillus amyloliquefaciens, Lactobacillus brevis, Lactobacillus delbrueckii, Lactobacillus reuteri, Lactobacillus helveticus* oder Gemischen davon ausgewählt ist; und einen Schritt eines Trocknens, um ein gliadinfreies Mehl zu erhalten.

2. Verfahren zum Abbau von Gliadin in Mehl zur Brotherstellung nach Anspruch 1, weiterhin **dadurch gekennzeichnet, dass** Pilzenzyme in dem enzymatischen Hydrolyseschritt, vorzugsweise Enzyme von *Aspergillus niger* und/oder *Aspergillus oryzae* in einem 1:1-Verhältnis verwendet werden.

3. Verfahren zum Abbau von Gliadin in Mehl zur Brotherstellung nach Anspruch 2, weiterhin **dadurch gekennzeichnet, dass** die Enzyme dem Gemisch in einem Anteil von zwischen 0,01 und 0,1 Gew.-% bezogen auf die Suspension zugegeben werden.

4. Verfahren zum Abbau von Gliadin in Mehl zur Brotherstellung nach Anspruch 1, weiterhin **dadurch gekennzeichnet, dass** der enzymatische Hydrolyseschritt bei einer Temperatur von zwischen 35 und 37 °C, vorzugsweise unter konstantem Rühren durchgeführt wird.

5. Verfahren zum Abbau von Gliadin in Mehl zur Brotherstellung nach Anspruch 1, weiterhin **dadurch gekennzeichnet, dass** die Hydrolysezeit von zwischen 4 und 8 Stunden beträgt.

6. Verfahren zum Abbau von Gliadin in Mehl zur Brotherstellung nach Anspruch 1, weiterhin **dadurch gekennzeichnet, dass**, nachdem der Hydrolyseschritt abgeschlossen ist, eine pH-Wert-Einstellung durchgeführt wird, vorzugsweise wobei der pH-Wert auf zwischen 6,5 und 8 eingestellt wird.

7. Verfahren zum Abbau von Gliadin in Mehl zur Brotherstellung nach Anspruch 6, weiterhin **dadurch gekennzeichnet, dass** die pH-Wert-Einstellung durch Zugeben einer Base, vorzugsweise K₂CO₃ durchgeführt wird.

8. Verfahren zum Abbau von Gliadin in Mehl zur Brotherstellung nach Anspruch 1, weiterhin **dadurch gekennzeichnet, dass** der verwendete Mikroorganismus dem Gemisch in einer Menge von zwischen 10³ und 10⁹ KBE/g Mehl zugegeben wird.

9. Verfahren zum Abbau von Gliadin in Mehl zur Brotherstellung nach Anspruch 1, weiterhin **dadurch gekennzeichnet, dass** der Fermentierschritt bei einer Temperatur von 36 bis 38 °C, vorzugsweise für einen Zeitraum von zwischen 4 und 8 Stunden durchgeführt wird.

10. Verfahren zum Abbau von Gliadin in Mehl zur Brotherstellung nach Anspruch 1, weiterhin **dadurch gekennzeichnet, dass**, nachdem der Fermentierschritt abgeschlossen ist, eine zweite pH-Wert-Einstellung durchgeführt wird, vorzugsweise wobei der pH-Wert auf zwischen 6,5 und 8 eingestellt wird.

11. Verfahren zum Abbau von Gliadin in Mehl zur Brotherstellung nach Anspruch 10, weiterhin **dadurch gekennzeichnet, dass** die zweite pH-Wert-Einstellung durch Zugeben einer Base, vorzugsweise K₂CO₃ durchgeführt wird.

12. Verfahren zum Abbau von Gliadin in Mehl zur Brotherstellung nach Anspruch 1, weiterhin **dadurch gekennzeichnet, dass** es einen zweiten enzymatischen Hydrolyseschritt umfasst.

13. Verfahren zum Abbau von Gliadin in Mehl zur Brotherstellung nach Anspruch 12, weiterhin **dadurch gekennzeichnet, dass** Pilzenzyme in dem zweiten enzymatischen Hydrolyseschritt, vorzugsweise Enzyme von *Aspergillus niger* und/oder *Aspergillus oryzae* in einem 1:1-Verhältnis verwendet werden.

14. Verfahren zum Abbau von Gliadin in Mehl zur Brotherstellung nach Anspruch 13, weiterhin **dadurch gekennzeichnet, dass** die Enzyme dem Gemisch in einem Anteil von zwischen 0,01 und 0,1 Gew.-% bezogen auf die Suspension zugegeben werden.

15. Verfahren zum Abbau von Gliadin in Mehl zur Brotherstellung nach Anspruch 13, weiterhin **dadurch gekennzeichnet, dass** der zweite enzymatische Hydrolyseschritt bei einer Temperatur von zwischen 35 und 37 °C, vorzugsweise unter konstantem Rühren durchgeführt wird.

16. Verfahren zum Abbau von Gliadin in Mehl zur Brotherstellung nach Anspruch 13, weiterhin **dadurch gekennzeichnet, dass** eine zweite enzymatische Hydrolysezeit von 2 bis 4 Stunden beträgt.

17. Verfahren zum Abbau von Gliadin in Mehl zur Brotherstellung nach Anspruch 1, weiterhin **dadurch gekennzeichnet, dass** es einen zweiten Fermentierschritt umfasst.

18. Verfahren zum Abbau von Gliadin in Mehl zur Brotherstellung nach Anspruch 17, weiterhin **dadurch gekennzeichnet, dass** der zweite Fermentierschritt durch Verwenden von mindestens einem Mikroorganismus unter kontrollierten pH-Wert-Bedingungen durchgeführt wird, vorzugsweise wobei der Mikroorganismus aus *Bacillusamyloliquefaciens-, Lactobacillus-brevis-, Lactobacillus-delbrueckii-, Lactobacillus-reuteri-,* Lactobacillus-helveticus-Mikroorganismen oder Gemischen davon ausgewählt ist.

19. Verfahren zum Abbau von Gliadin in Mehl zur Brotherstellung nach Anspruch 18, weiterhin **dadurch gekennzeichnet, dass** ein Gemisch von Bacillus amyloliquefaciens und mindestens zwei Laktobakterien, ausgewählt aus: *Lactobacillus brevis, Lactobacillus delbrueckii, Lactobacillus reuteri* und *Lactobacillus helveticus,* vorzugsweise in einem 1:1:1 -Verhältnis verwendet wird.

20. Verfahren zum Abbau von Gliadin in Mehl zur Brotherstellung nach Anspruch 19, weiterhin **dadurch gekennzeichnet, dass** der Mikroorganismus dem Gemisch in einer Menge von zwischen 10³ und 10⁹ KBE/g Mehl zugegeben wird.

21. Verfahren zum Abbau von Gliadin in Mehl zur Brotherstellung nach Anspruch 18, weiterhin **dadurch gekennzeichnet, dass** der zweite Fermentierschritt bei einer Temperatur von zwischen 35 und 37 °C, vorzugsweise unter konstantem Rühren durchgeführt wird.

22. Verfahren zum Abbau von Gliadin in Mehl zur Brotherstellung nach Anspruch 19, weiterhin **dadurch gekennzeichnet, dass** der zweite Fermentierschritt für einen Zeitraum von zwischen 4 und 12 Stunden durchgeführt wird.

23. Verfahren zum Abbau von Gliadin in Mehl zur Brotherstellung nach Anspruch 1, weiterhin **dadurch gekennzeichnet, dass** der Trocknungsschritt ein Sprühtrocknungsverfahren ist.

24. Verfahren zum Abbau von Gliadin in Mehl zur Brotherstellung nach Anspruch 23, weiterhin **dadurch gekennzeichnet, dass** Zuluft bei einer Temperatur von zwischen 150 und 180 °C ist und Abluft bei einer Temperatur von zwischen 70 und 90 °C in dem Sprühtrocknen ist.

## Revendications

1. Procédé pour la dégradation de la gliadine dans la farine pour faire du pain, **caractérisé en ce qu'**il comprend : une étape de mélange de la farine avec de l'eau ; au moins une étape d'hydrolyse enzymatique pour hydrolyser la gliadine à partir du mélange obtenu dans l'étape de mélange ; au moins une étape de fermentation du mélange obtenu dans l'étape d'hydrolyse enzymatique par utilisation d'au moins un microorganisme dans des conditions à pH contrôlé ; dans lequel le microorganisme est choisi parmi *Bacillus amyloliquefaciens, Lactobacillus brevis, Lactobacillus delbrueckii, Lactobacillus reuteri, Lactobacillus helveticus* et leurs mélanges ; et une étape de séchage pour obtenir une farine sans gliadine.

2. Procédé pour la dégradation de la gliadine dans la farine pour faire du pain selon la revendication 1, **caractérisé en outre en ce que** des enzymes fongiques sont utilisées dans l'étape d'hydrolyse enzymatique, de préférence des enzymes provenant *d'Aspergillus niger* et/ou *Aspergillus oryzae* en un rapport de 1/1.

3. Procédé pour la dégradation de la gliadine dans la farine pour faire du pain selon la revendication 2, **caractérisé en outre en ce que** les enzymes sont ajoutées au mélange en une proportion comprise entre 0,01 et 0,1 % en poids par rapport à la suspension.

4. Procédé pour la dégradation de la gliadine dans la farine pour faire du pain selon la revendication 1, **caractérisé en outre en ce que** l'étape d'hydrolyse enzymatique est effectuée à une température comprise entre 35 et 37°C, de préférence sous agitation constante.

5. Procédé pour la dégradation de la gliadine dans la farine pour faire du pain selon la revendication 1, **caractérisé en outre en ce que** le temps d'hydrolyse est compris entre 4 et 8 heures.

6. Procédé pour la dégradation de la gliadine dans la farine pour faire du pain selon la revendication 1, **caractérisé en outre en ce qu'**après la fin de l'étape d'hydrolyse, un ajustement du pH est effectué, le pH étant de préférence ajusté entre 6,5 et 8.

7. Procédé pour la dégradation de la gliadine dans la farine pour faire du pain selon la revendication 6, **caractérisé en outre en ce que** l'ajustement du pH est effectué par addition d'une base, de préférence K₂CO₃.

8. Procédé pour la dégradation de la gliadine dans la farine pour faire du pain selon la revendication 1, **caractérisé en outre en ce que** le microorganisme utilisé est ajouté au mélange en une quantité comprise entre 10³ et 10⁹ UFC/g de farine.

9. Procédé pour la dégradation de la gliadine dans la farine pour faire du pain selon la revendication 1, **caractérisé en outre en ce que** l'étape de fermentation est effectuée à une température de 36 à 38°C, de préférence pendant une période comprise entre 4 et 8 heures.

10. Procédé pour la dégradation de la gliadine dans la farine pour faire du pain selon la revendication 1, **caractérisé en outre en ce qu'**après la fin de l'étape de fermentation, un deuxième ajustement du pH est effectué, le pH étant de préférence ajusté entre 6,5 et 8.

11. Procédé pour la dégradation de la gliadine dans la farine pour faire du pain selon la revendication 10, **caractérisé en outre en ce que** le deuxième ajustement du pH est effectué par addition d'une base, de préférence K₂CO₃.

12. Procédé pour la dégradation de la gliadine dans la farine pour faire du pain selon la revendication 1, **caractérisé en outre en ce qu'**il comprend une deuxième étape d'hydrolyse enzymatique.

13. Procédé pour la dégradation de la gliadine dans la farine pour faire du pain selon la revendication 12, **caractérisé en outre en ce que** des enzymes fongiques sont utilisées dans la deuxième étape d'hydrolyse enzymatique, de préférence des enzymes provenant *d'Aspergillus niger* et/ou *Aspergillus oryzae* en un rapport de 1/1.

14. Procédé pour la dégradation de la gliadine dans la farine pour faire du pain selon la revendication 13, **caractérisé en outre en ce que** les enzymes sont ajoutées au mélange en une proportion comprise entre 0,01 et 0,1 % en poids par rapport à la suspension.

15. Procédé pour la dégradation de la gliadine dans la farine pour faire du pain selon la revendication 13, **caractérisé en outre en ce que** la deuxième étape d'hydrolyse enzymatique est effectuée à une température comprise entre 35 et 37°C, de préférence sous agitation constante.

16. Procédé pour la dégradation de la gliadine dans la farine pour faire du pain selon la revendication 13, **caractérisé en outre en ce que** le temps de la deuxième hydrolyse enzymatique est de 2 à 4 heures.

17. Procédé pour la dégradation de la gliadine dans la farine pour faire du pain selon la revendication 1, **caractérisé en outre en ce qu'**il comprend une deuxième étape de fermentation.

18. Procédé pour la dégradation de la gliadine dans la farine pour faire du pain selon la revendication 17, **caractérisé en outre en ce que** la deuxième étape de fermentation est effectuée par utilisation d'au moins un microorganisme dans des conditions à pH contrôlé, le microorganisme étant de préférence choisi parmi les microorganismes *Bacillus amyloliquefaciens, Lactobacillus brevis, Lactobacillus delbrueckii, Lactobacillus reuteri, Lactobacillus helveticus* ou leurs mélanges.

19. Procédé pour la dégradation de la gliadine dans la farine pour faire du pain selon la revendication 18, **caractérisé en outre en ce qu'**un mélange de *Bacillus amyloliquefaciens* et d'au moins deux lactobacilles choisis parmi *Lactobacillus brevis, Lactobacillus delbrueckii, Lactobacillus reuteri* et *Lactobacillus helveticus* est utilisé, de préférence en un rapport de 1/1/1.

20. Procédé pour la dégradation de la gliadine dans la farine pour faire du pain selon la revendication 19, **caractérisé en outre en ce que** le microorganisme est ajouté au mélange en une quantité comprise entre 10³ et 10⁹ UFC/g de farine.

21. Procédé pour la dégradation de la gliadine dans la farine pour faire du pain selon la revendication 18, **caractérisé en outre en ce que** la deuxième étape de fermentation est effectuée à une température comprise entre 35 et 37°C, de préférence sous agitation constante.

22. Procédé pour la dégradation de la gliadine dans la farine pour faire du pain selon la revendication 19, **caractérisé en outre en ce que** la deuxième étape de fermentation est effectuée pendant une période comprise entre 4 et 12 heures.

23. Procédé pour la dégradation de la gliadine dans la farine pour faire du pain selon la revendication 1, **caractérisé en outre en ce que** l'étape de séchage est un procédé de séchage par pulvérisation.

24. Procédé pour la dégradation de la gliadine dans la farine pour faire du pain selon la revendication 23, **caractérisé en outre en ce que** l'air d'entrée est à une température comprise entre 150 et 180°C et l'air de sortie est à une température comprise entre 70 et 90°C dans le séchage par pulvérisation.
